(19) **Europäisches Patentamt / European Patent Office / Office européen des brevets**

(11) **EP 2 582 293 B1**

(12) # EUROPEAN PATENT SPECIFICATION

(45) Date of publication and mention of the grant of the patent:
**20.04.2016 Bulletin 2016/16**

(21) Application number: **11743656.8**

(22) Date of filing: **20.06.2011**

(51) Int Cl.:
**A61B 5/103** (2006.01)     **A61B 5/00** (2006.01)

(86) International application number:
**PCT/NL2011/000051**

(87) International publication number:
**WO 2011/159148 (22.12.2011 Gazette 2011/51)**

(54) **METHOD AND APPARATUS FOR DETERMINING AN AUTOFLUORESCENCE VALUE OF SKIN TISSUE.**

VERFAHREN UND VORRICHTUNG ZUR BESTIMMUNG EINES AUTOFLUORESZENZWERTES VON HAUTGEWEBE

PROCÉDÉ ET APPAREIL PERMETTANT DE DÉTERMINER LA VALEUR D'AUTOFLUORESCENCE D'UN TISSU CUTANÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2010 NL 2004920**

(43) Date of publication of application:
**24.04.2013 Bulletin 2013/17**

(73) Proprietor: **DiagnOptics Holding B.V.**
**9723 JJ Groningen (NL)**

(72) Inventors:
• **GRAAFF, Reindert**
**NL-9736 CN Groningen (NL)**
• **KOETSIER, Marten**
**9723 JJ Groningen (NL)**
• **SMIT, Andries, Jan**
**NL-9725 KM Groningen (NL)**
• **VAN DEN BERG, Bartholomeus, Adrianus**
**NL-2012 BA Haarlem (NL)**
• **VAN DER ZEE, Pieter**
**NL-9746 PV Groningen (NL)**

(74) Representative: **V.O.**
**P.O. Box 87930**
**2508 DH Den Haag (NL)**

(56) References cited:
**WO-A1-01/22869        WO-A2-2005/045393
WO-A2-2009/086474     JP-A- 2009 247 430
US-A1- 2007 276 199**

**Description**

**FIELD AND BACKGROUND OF THE INVENTION**

The invention relates to a method for determining an autofluorescence (AF) value of skin tissue of a subject according to the introductory portion of claim 1 and to an apparatus for determining an autofluorescence (AF) value of skin tissue of a subject according to the introductory portion of claim 14. Such a method and such an apparatus are known from WO01/22869.

Measuring skin AF is a non-invasive method for determining the amount of accumulated tissue Advanced Glycation Endproducts (AGEs). A significant correlation exists between skin AF and levels of skin AGEs like pentosidine, $N\varepsilon$-carboxy-methyllysine (CML) and $N\varepsilon$-carboxy-ethyllysine (CEL), as obtained from skin biopsies: in a combined analysis of skin biopsy validation studies, 76% of the variation in skin AF was explained by variations in skin biopsy pentosidine levels [Meerwaldt 2004, 2005, den Hollander 2007] (see the listing of references cited after the detailed description). Skin AF has been shown to increase with age and is also an independent predictor of development and progression of complications in diabetes mellitus, renal failure and other diseases with increased cardiovascular risk [Meerwaldt 2005, Mulder 2008, Lutgers 2009, Matsumoto 2007, Ueno 2008, Monami 2008]. Skin AF can for instance be measured with an optical measurement instrument such as AGE Reader (DiagnOptics Technologies BV, Groningen, The Netherlands, cf. international patent application WO 01/22869 ), from the mean emission in the 420 - 600 nm range upon UV-A excitation (i.e. within a range of approximately 315 - 400 nm) with a peak wavelength of 370 nm. Further methods for skin AF determination are disclosed in international patent application WO2005/045393.

It has been shown that skin AF measurements in subjects with darker skin colors (UV-reflectance below 10%) typically result in lower values than in subjects with fair skin colors [Mulder 2006]. It is not expected that these subjects have a substantially lower amount of AGEs. The lower AF values are therefore expected to be caused by different absorption of excitation or emission light by skin compounds and scattering effects, especially in the epidermis, and specular reflectance. The observed skin color dependence hinders reliable assessment of skin AGEs in subjects with darker skin color and inhibits the recognition of increased skin AF values.

Literature provides some methods to describe the influence of absorbers and scatterers on skin color [Kollias 1987, Nishidate 2004, Zonios 2006, Sandby-Møller 2003].

A problem of skin AF measurements on subjects with darker skin colors is thus that typically lower AF values are measured than on subjects with fair skin colors having the same AGE level. This means that, for persons of different skin colors, skin AF values as determined in the known manner is not a reliable basis for estimating a person's skin AGE level and thus also not for predicting that person's associated health risk. This makes the known technique not generally applicable to subjects of various skin colors.

To compensate for differences in skin color, skin AF was initially calculated as the mean light intensity in the emission range divided by the mean light intensity of the light that is reflected from tissue in the excitation range, as suggested previously by Coremans et al. [Coremans 1997]. Whenever more melanin or other skin compounds are absorbing emission light, they also absorb more excitation light and by dividing these two quantities, the result will be less dependent on absorption. Using this method, skin AF can reliably be obtained in subjects with Fitzpatrick skin phototypes I - IV. Stamatas et al. [Stamatas 2006] also used the reflectance of the skin as a normalization factor for AF measurements. They also reported that this method is adequate, but only for lighter skin types. In the AGE Reader, a simple skin color assessment is performed using the mean intensity of the UV-A light that is reflected from the skin. It was found that skin AF can be reliably assessed if more than 10% of the UV-A light is reflected [Mulder 2006, Koetsier 2010]. This method could not compensate for the strong absorption of melanin, as in subjects with a dark skin color.

In the name AGE Reader, the excitation light source illuminates in the 350 - 410 nm range and emission is measured in the 420 - 600 nm range. Skin AF in these ranges may not only be caused by skin AGEs. Also other fluorophores such as keratin, vitamin D, lipofuscin, ceroid, NADH and pyridoxine may add to the total fluorescence signal [Bachmann 2006]. Furthermore, some fluorophores have excitation maxima that are within the emission range of the fluorophores above, including porphyrins, elastin crosslinks, FAD, flavins and phospholipids. Due to the overlapping nature of absorption and emission spectra, it is difficult, if not impossible, to assess the influence of specific fluorophores on the total fluorescence signal, especially with the broad excitation peak that is used in the AGE Reader. However, it has been shown that even with this broad excitation peak, dermal content of specific AGEs explains the major part of the variance (up to 76%) in the skin AF signal in a pooled analysis of the validation studies mentioned earlier, and, moreover, that the risk of chronic complications in diabetes can be assessed [Koetsier 2009].

Apart from other fluorophores, non-fluorescent chromophores in the skin may have an effect on skin AF by selectively absorbing excitation and/or emission light. The most contributory chromophores in the UV-A and visible region are melanin in the epidermis and hemoglobin in the dermis [Anderson 1981, Sinichkin 2002, Kollias 2002]. Both in the epidermis and the dermis, also bilirubin and to a lesser extent beta-carotene are present, having absorption peaks at 470 nm and 450 nm respectively [Anderson 1981, Bachmann 2006]. Nevertheless, melanin and hemoglobin are widely accepted as the main absorbers.

The absorption spectrum of melanin has been studied extensively in vitro [Zonios 2008a]. However, melanin resides in the skin in cell organelles, melanosomes, and the effect on skin color and moreover on the measurement of AF is influenced by the size, number, distribution and aggregation of these melanosomes in the skin, which may vary largely between individuals of different ethnic groups [Alaluf 2002, Barsh 2003]. In general, melanin absorbs light from the UV, visible and near infrared range of the spectrum, with an exponential increase of absorption towards lower wavelengths [Zonios 2008a, Zonios 2008b].

Hemoglobin has a broad absorption spectrum over the visual part of the spectrum with several absorption peaks and is therefore an important factor in skin color [Anderson 1981, Feather 1989, Bachmann 2006]. Although it is not expected that the hemoglobin concentration or distribution is very different for the various skin phototypes, the apparent optical properties of hemoglobin and their influence on skin AF may vary because of interactions with other chromophores (e.g. melanin) during light propagation within the skin. Moreover, hemoglobin is concentrated in red blood cells within blood vessels. Because of a limited and wavelength dependent penetration depth of light in blood vessels, the influence of hemoglobin on skin AF is difficult to assess. Nevertheless, Na et al. observed a variation of skin AF in their measurements as a function of skin redness, which depends on hemoglobin concentration or oxygen saturation [Na 2001].

Several approaches exist to describe the influence of absorbers and scatterers on skin color. Some methods have used a homogeneous approach [Kollias 1987, Sinichkin 2002, Nishidate 2004, Zonios 2006, Sandby-Møller 2003], whereas others have defined many layers in the skin, with separate optical properties in each layer, that may vary between subjects [Magnain 2007, Nielsen 2008, Katika 2006, Chen 2007]. Some of these approaches aim at determining the concentration of certain chromophores or identifying specific fluorophores.

## SUMMARY OF THE INVENTION

It is an object of the invention to assess skin fluorescence more independently of skin characteristics and to provide a solution for adapting the measured skin AF for the influence of skin color. For that purpose the invention provides a method according to claim 1. The invention can also be embodied in an apparatus according to claim 14, which is specifically adapted for carrying out a method according to claim 1.

According to the invention, the determined AF value is obtained by correcting the measured AF value , in such manner that the dependency of the determined AF value upon different UV-skin tissue reflectances, that different respective subjects may have, is minimized or at least diminished. This minimizing or at least diminishing of said dependency makes the technique according to the invention applicable to subjects of various skin characteristics.

Particular embodiments of the invention are set forth in the dependent claims.

Further considerations, details, aspects and embodiments of the invention are described, by way of non-limiting example only in the following detailed description.

BRIEF DESCRIPTION OF THE DRAWINGS

Fig. 1 is a graph of intensity spectra of an UV blacklight tube and of a white LED used for illuminating the skin of subjects;
Fig. 2 is a graph of typical reflectance spectra of three subjects with values of UV-reflectance of 4.4%, 8.0% and 11.4% respectively;
Fig. 3 is a graph of typical emission spectra of three subjects with values of UV-reflectance of 4.4%, 8.0% and 11.4% respectively;
Fig. 4 shows part of the normalized reflectance spectra as measured with the AGE Reader from healthy subjects with light and dark skin color;
Fig. 5 is a graph of adjusted skin AF as a function of UV-reflectance comparing the preferred new algorithm (b) and the old method for calculating skin AF (a);
Fig. 6 is a graph of the AF values as a function of subject age, as calculated with the new algorithm (b) as well as without correction for skin color (a);
Fig. 7 is a schematic representation of a first example of an apparatus according to the invention; and
Fig. 8 is a schematic representation of a second example of an apparatus according to the invention.

## DETAILED DESCRIPTION

### Introduction

Various parameters from the spectra have been formulated for the purpose of obtaining parameters correlating with and thus predictive for the decrease of the measured AF for darker skin colors. With these parameters, multiple linear regression analysis was performed to determine how the formulated parameters relate to the deviation of the measured AF from an AF value corrected for skin color. Based on this model, a preferred algorithm and alternatives to calculate a corrected skin AF have been constructed and subsequently validated using measurements on healthy subjects of various skin color.

Below, examples of a method and a device according to the invention are described and the performance of some embodiments with respect to the correction of AF measurements is described.

### Materials and methods

### *Measurement setup*

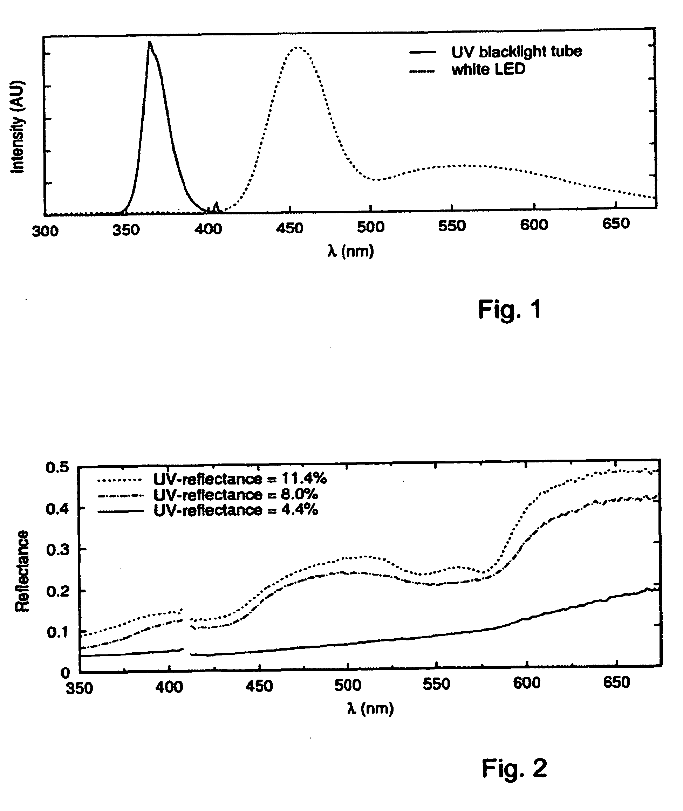

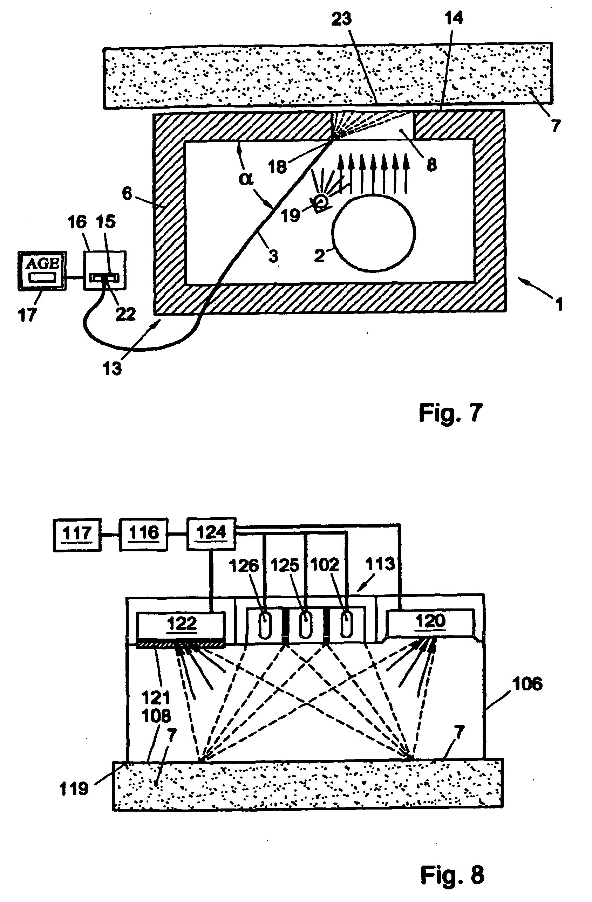

Skin AF was measured with an AGE Reader as shown in Fig. 7. The measuring system 1 shown in Fig. 7 comprises a measuring unit 13 having as a light source a fluorescent lamp in the form of an UV-A blacklight tube 2 (F4T5BLB, Philips, Eindhoven, The Netherlands), with a peak wavelength of 370 nm. The lamp 2 is arranged within a supporting structure in the form of a light-shielding casing 6. The casing 6 has a contact surface 14 against which the volar side of the forearm 7 of the subject is placed to illuminate a surface 23 of~4 cm$^2$ of the skin on the volar side of the forearm. A spectrum of the light source 2 is shown in Figure 1. For determining reflective properties of the skin in the autofluorescent wavelength range (i.e. outside the wavelength range of the excitation radiation) a second light source 19 in the form of a white LED is provided. A spectrum of this light source 19 is shown in Figure 1 as well. Located adjacent an edge of the irradiation window 8 is an end 18 of a non-contact optical fiber 3 (200 $\mu$m diameter) for receiving the autofluorescent emission and reflected excitation light from a skin surface of ~0.4 cm$^2$ at an angle a of 45°. Via the optical fiber, radiation to be detected, received from the skin 7, is passed to a spectrometer 15 (AvaSpec_2048, Avantes, Eerbeek, The Netherlands) with an array 22 of detectors. A computer 16 is programmed with computer software for analyzing the intensity spectrum analyzed and for generating signals representing an AGE content in the skin 7 on a display 17.The software loaded into the computer provides, by means of the display 17, for the generation of a signal which represents a measured AF in agreement with the measured amount of electromagnetic radiation in the wavelength range outside the wavelength range of the radiation applied to the skin 7. According to this example, the software is further designed for processing the amount of electromagnetic radiation, measured via measuring window 18, in the wavelength range within the wavelength range of the radiation applied to the skin 7, for the purpose of correcting for the optical properties of the skin tissue in accordance with methods described below.

It is noted that the use of a spectrometer provides the advantage that it can be accurately determined per narrow wavelength band to what extent it is being taken into account as an indicator of the presence of AGE's. However, a more compact and more easily portable apparatus which is also more suitable to be held against a patient's body at different locations can be provided as well. In the considerably smaller measuring unit 113 according to the example shown in fig. 8, as an excitation radiation source, a LED 102 is provided which, according to this example, emits radiation of a wavelength of about 370 nm, or at least in a range of 300-420 nm, preferably only in a narrow band (width at half of the highest intensity, for instance, 10 nm).

A second LED 125 is arranged for emitting only light of wavelength in a range higher than 620 nm or 625 nm and preferably not higher than 900 or 880 nm to the skin 7. A third LED 126 is arranged for emitting only light of wavelength in a range from 450 nm to 525 nm and preferably around 500 nm to the skin 7.

LED's are easy to control in a pulsed or modulated fashion, which is advantageous for correcting, for instance, for dark current due of the detector 122 or ambient light. The measuring unit 113 has a screening 106 for screening off ambient light and an irradiation window 108 having a limiting edge 119 to be placed against the skin 7.

For detecting radiation coming from the skin 7, two detectors 120, 122 are used which can simultaneously detect radiation coming from the skin 7 and are arranged to be coupled to a computer for analysis of the. Arranged between the detector 122 and the skin 7 is a long pass filter 121, which passes only radiation of a wavelength greater than, for instance, 400 nm, so that the detector 122 only receives radiation from the skin 7 in the fluorescence-induced wavelength range. The detector 120 is preferably arranged for detecting the total amount of light arriving from the skin 7 at the accumulated wavelength ranges of the LEDs 102, 125, 126.

The LEDs 102, 125, 126 and the detectors 120, 122 are connected to a control unit 124 coupled to a computer 116 with a display 117. The control unit 124 is arranged to activate and deactivate the LEDs and to output detected radiation values received from the decteors 120, 122 under control of the computer 116.

The LEDs can be activated sequentially to generate successively generate at different wavelengths or wavelength ranges and to measure the reflectance at different wavelengths or wavelength ranges as well as the AF without using a spectrometer. Operation of the LEDs will be discussed below in the context of the described examples of methods according to the invention.

Instead of two detectors, it is also possible to provide, for instance, a single detector and a chopper which passes alternately radiation of all wavelengths and radiation solely above a particular wavelength. This provides the advantage that measuring errors as a result of differences between the two detectors are prevented, but leads to an increase of the dimensions and the mechanical complexity of the measuring unit.

Instead of different light sources for measuring reflectance at different wavelengths or wavelength ranges it is also possible to provide different detectors for measuring reflectance at different wavelengths or wavelength ranges. Also, a plurality of detectors for detecting light at the same wavelength range can be used, for instance placed at different distances from the skin and (parallel to the skin) from the radiation source.

In the examples shown above, the detectors and the light capturing optical fiber are arranged spaced from the skin. However, the detectors and/or optical fiber(s) may also be arranged to be in contact with the skin when the pick-up unit is placed against the skin.

The value of the measured skin AF is calculated as the ratio between the total emission intensity (420 - 600 nm) and the total excitation intensity (300 - 420 nm), multiplied by 100 and is expressed in arbitrary units (AU). Besides the skin AF measurement, UV-reflectance is calculated as the sum of the intensities of the reflected light from the skin in the range 300-420 nm, divided by the sum of intensities in the same range from a white reference standard, which is embedded in the AGE Reader and has been calibrated in situ against an external reflectance standard. Moreover, a complete diffuse reflectance spectrum is obtained, using a white LED as illumination source in the visible range. This LED is located directly under the detecting fiber. The spectrum of the LED is also shown in Figure 1. All spectra were corrected for dark current and stored in a file for later analysis.

### Subjects

Three cohorts of healthy subjects were used in this study. The first group consisted of 61 subjects of Afro-Caribbean descent with a negroid dark skin color, living in the Netherlands. The second group was a group of 120 southern Chinese subjects with intermediate skin color, living in China. The third group consisted of 60 subjects of Asian and African descent, all living in the Netherlands. Health status was obtained by clinical assessment (first and second cohorts) or using a self-administered questionnaire (third cohort). For all these cohorts, only subjects with a UV-reflectance below 12% and with subject age between 20 and 70 years were included. Subjects were excluded if not all spectra were obtained correctly.

For assessing correlations between age-corrected skin AF and various parameters that were derived from reflectance spectra in the UV-A and visible range, a subset of 99 subjects (33 subjects from each cohort) was chosen from the total group. The selection focused on obtaining a group of subjects over the full range of age (20 - 70 years) and UV-reflectance values (~3% - 12%), and was otherwise random. For the validation, all other subjects were used (N = 142).

### Model outline

The corrected AF values obtained using new correction methods according to the invention and the preferred new algorithm have been compared with an existing model that describes the deviation of the measured AF from an expected value. The expected AF of an individual can be described as a function of subject age in years, with AF = 0.024 × age + 0.83. This relation is based on a large set of Caucasian healthy persons with a UV-reflectance value above 10% [Koetsier 2010]. With this, the deviation of skin AF for a particular individual is calculated as

$$\Delta AF = AF_m - AF(age) = AF_m - 0.024 \times age - 0.83, \qquad (1)$$

where $AF_m$ is the skin AF as measured. $\Delta AF$ was used as the dependent variable in the fitting model.

### Signals and data processing

Parameters that describe the skin color and can be measured with the AGE Reader can relate to two types of spectra that are available. First, the spectrum that is measured directly from the skin during illumination with the UV light source. This spectrum includes a large peak of UV light that is reflected from the skin and a small emission peak, due to AF of the AGEs and possibly also other skin compounds with fluorescence emission in the same wavelength region, such as NADH and lipofuscins. Secondly, a reflectance spectrum is available that represents the relative skin reflectance as compared to a white reference standard. This spectrum consists of two parts, one measured with the UV light source, $\sim$ 350 - 410 nm, and one measured with a white light source, $\sim$ 415 - 675 nm. The parameters were based on literature study and own observations.

It is not known whether the parameters as calculated from the spectra are independent of subject age. Therefore, also subject age was included in the model, to compensate for possible interactions.

The parameters were assessed for normality and collinearity using SPSS (version 16, SPSS Inc., Chicago, IL). Parameters were considered normally distributed if a Kolmogorov-Smirnov test resulted in a p-value above 0.05. Parameters were considered independent if the tolerance level exceeded 0.01. For the backward multivariate analysis, threshold p-values of 0.01 and 0.05 were considered.

### *Principle of the algorithm*

With the formulated parameters, a prediction model for $\Delta$AF was obtained, using a backward multiple linear regression analysis. Since the average expected $\Delta$AF of any group of healthy subjects is assumed to be zero, the predicted $\Delta$AF, $\Delta AF_{pred}$, was then used as a correction for $AF_m$ as

$$\mathbf{AF_{corr} = AF_m - \Delta AF_{pred}.} \qquad\qquad (2)$$

### *Validation*

Since the low skin AF values were first observed in subjects that had a UV-reflectance below 10%, the derived algorithm for calculating skin AF can be validated by describing skin AF as a function of the UV-reflectance. For this, age-corrected skin AF(age), $\Delta AF_{corr} = AF_{corr}$ - AF(age), is used. Requirements are that $\Delta AF_{corr}$ should not be dependent on UV-reflectance and mean $\Delta AF_{corr}$ should be close to zero. Furthermore, the increase of skin AF values with subject age should match the reference values that were found earlier [Koetsier 2010].

### Results

### *Subjects*

Table 1 summarizes group size, skin color and age characteristics of the three cohorts separately and as a whole, for the model development group and the validation group separately. UV-reflectance was used as a measure of skin color. In the first group (subjects of Afro-Caribbean descent), one subject was excluded because of an artifact in one of the spectra.

**Table 1.** Group characteristics of the datasets used.

| group | size | UV-reflectance (%) | | Age (years) | |
|---|---|---|---|---|---|
| | | mean $\pm$ sd | range | mean $\pm$ sd | range |
| Measurements used for model development | | | | | |
| group 1 (AC) | 33 | 4.59 $\pm$ 1.36 | 2.55 - 7.99 | 41.5 $\pm$ 11.5 | 20 - 69 |
| group 2 (SC) | 33 | 9.16 $\pm$ 1.54 | 6.69 - 11.79 | 40.4 $\pm$ 15.8 | 21 - 70 |
| group 3 (VO) | 33 | 8.49 $\pm$ 2.21 | 4.13 - 11.53 | 40.9 $\pm$ 12.8 | 20 - 69 |
| Total | 99 | 7.41 $\pm$ 2.66 | 2.55 - 11.79 | 40.9 $\pm$ 13.3 | 20 - 70 |
| Measurements used for validation | | | | | |
| group 1 (AC) | 27 | 5.32 $\pm$ 1.68 | 3.20 - 10.40 | 41.6 $\pm$ 13.5 | 20 - 70 |
| group 2 (SC) | 87 | 8.61 $\pm$ 1.84 | 4.30 - 11.55 | 46.8 $\pm$ 11.3 | 24 - 69 |
| group 3 (VO) | 27 | 9.03 $\pm$ 2.23 | 4.20 - 11.68 | 33.7 $\pm$ 10.4 | 20 - 58 |
| Total | 141 | 8.06 $\pm$ 2.31 | 3.20 - 11.68 | 43.3 $\pm$ 12.6 | 20 - 70 |

*Groups consisted of Afro-Caribbean (AC) and southern Chinese (SC) subjects and subjects of various origin (VO).*

*Parameters for prediction of ΔAF*

Proposed parameters that may predict the deviation of AF from an expected value, ΔAF, are described below. Most parameters are related to melanin, hemoglobin or bilirubin, since these are the strongest absorbers in the skin. The parameters were analyzed for correlations using the dataset of 99 subjects. Table 2 summarizes the predictive properties of the parameters analyzed.

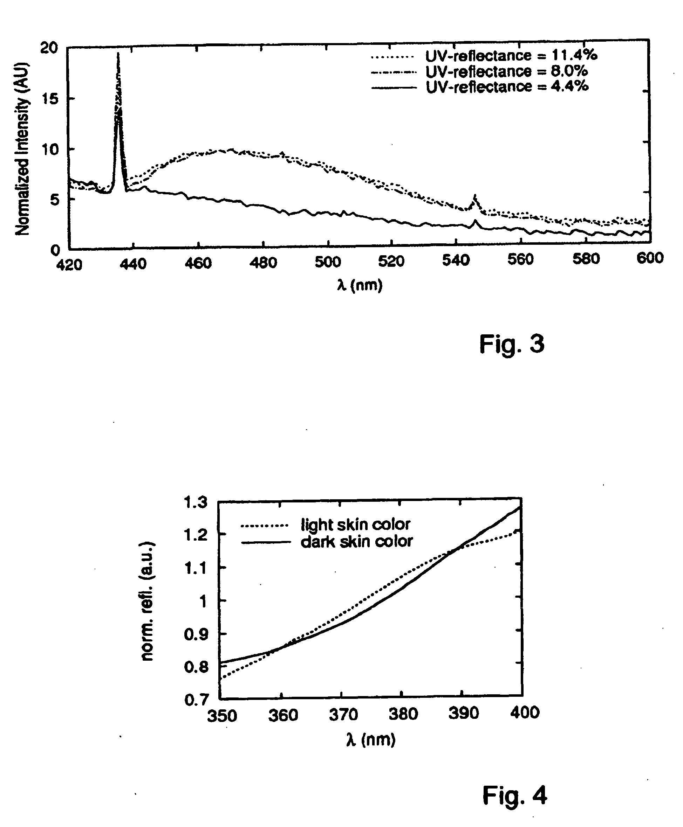

Figures 2 and 3 show typical reflectance and, respectively, emission spectra of three subjects with values of UV-reflectance of 4.4%, 8.0% and 11.4% respectively. The reflectance spectra show some distinctive features, that are believed to be caused by absorption of melanin, hemoglobin and other chromophores. The intensity of these features varies between subjects and this information is used for formulating the various parameters described below. As expected, the average reflectance of a subject with a dark skin color is lower. Figure 3 shows that the measured emission intensity is lower for subjects with darker skin color as well.

Reflectance in UV range

Since the start of the development of the AGE Reader, the UV-reflectance has been used as an indication of skin color. With this value, it was found that skin AF is lower than expected in subjects with darker skin colors, but no linear relation with ΔAF was found [Mulder 2006]. The inverse value of the UV-reflectance (InvRef1) was proposed and found to be linearly related to ΔAF. InvRef1 was used as a parameter in the model, not the UV-reflectance itself.

Melanin related parameters

The amount of melanin may be expressed as an index. Sinichkin et al. [Sinichkin 2002] provided three wavelength ranges in which the ratio between the reflectance at two wavelengths (or the slope in a logarithmic spectrum) can be used to determine this index.

First, the UV-A wavelength, because of the high absorption of melanin in UV. The suggested wavelength range is from 365 - 395 nm. In the AGE Reader, the UV light source is illuminating in this range.

However, in our measurements it was found that using 5 nm lower wavelengths yielded a better correlation with ΔAF. This wavelength range is centered around the peak wavelength of the light source used. The first melanin index (MI) parameter was defined as

$$MI_1 = R_{390} / R_{360}, \tag{3}$$

where R is reflectance and the subscripts denote the wavelength in nm.

MI may also be derived from the near infrared region, where hemoglobin absorption is relatively small. Kollias and Baqer used wavelengths up to 720 nm [Kollias 1985]. However, the white light source in the AGE Reader does not allow for this range, therefore, wavelengths up to 675 nm were used. Since two references [Kollias 1985, Dawson 1980] used different starting wavelengths, both pairs 620 - 675 nm and 650 - 675 nm were used in our study:

$$MI_2 = 100 \times (OD_{650} - OD_{675}) \tag{4}$$

$$MI_3 = 100 \times (OD_{620} - OD_{675}) \tag{5}$$

where $OD_\lambda$ is the apparent optical density at wavelength $\lambda$, defined as $- \log R_\lambda$.

While Sinichkin et al. proposed these wavelength pairs as ratios in the OD spectrum, Kollias and Baqer used a regression through the spectrum instead. This method is less prone to artifacts because it does not rely on just two values in the reflectance spectrum. Therefore, we introduced another parameter, RedLnSlope, representing the slope of the regression line through the spectrum of Ln(R) in the range 630 - 675 nm, multiplied by 100.

With more melanin, the melanin absorption causes a stronger decrease in the total reflectance spectrum, especially in the UV-range where melanin is the most important absorber. Figure 4 shows part of the normalized reflectance spectra as measured with the AGE Reader from healthy subjects with light and dark skin color. Both lines represent the average reflectance from six subjects, which were selected for having similar UV-reflectance values (approximately 18% for light skin color and approximately 6% for dark skin color). The shape of the spectrum of the subjects with light skin color appeared convex, whereas that of subjects with dark skin color showed to be concave. This shape can be quantified by assuming a line in the spectrum from the reflectance at 360 nm to the reflectance at 390 nm and then observing the deviation of the reflectance at 375 nm from the line. The deviation $UV_{shape}$ of the shape from a straight line was defined as

$$UV_{shape} = (R_{360} + R_{390}) / (2 \times R_{375}). \qquad (6)$$

No correlation was found between $UV_{shape}$ and $\Delta AF$ for subjects with darker skin colors ($R^2 = 0.077$). However, a linear correlation was found between $UV_{shape}$ and $R_{390}$, the reflectance at 390 nm, showing that $UV_{shape}$ is indeed dependent on skin color. With this correlation ($R^2 = 0.35$), a deviation was calculated per measurement, as a function of $UV_{shape}$ and $R_{390}$:

$$dUV_{shape} = UV_{shape} + 0.407 \times R_{390} - 1.036. \qquad (7)$$

This deviation value $dUV_{shape}$ was found to correlate linearly with $\Delta AF$ and was used as a parameter.

Furthermore, absolute reflectance values may be correlated to $\Delta AF$. In order to avoid interaction with hemoglobin, wavelengths had to be used where hemoglobin absorption is relatively low. Although no large differences in oxygen saturation were expected in healthy subjects, influence of oxygen saturation can easily be avoided by using isobestic points, where oxygenated and de-oxygenated hemoglobin have equal absorption.

Reflectance at the hemoglobin absorption minimum and isobestic point around 500 nm was first assessed. A linear correlation with $\Delta AF$ was found after a logarithmic transformation. The transformed parameter is referred to as $LnR_{500}$.

Finally, RedRefl was introduced as the mean reflectance in the 620-675 nm range.

Hemoglobin related parameters

Erythema is a condition where the apparent influence of hemoglobin in the skin is increased. Sinichkin et al. [Sinichkin 2002] have summarized the mostly used parameters that assess erythema as an index (EI), using reflectance spectra. These indices can be used to describe the influence of hemoglobin on skin AF values. Two different methods to describe erythema have been used. The first was based on the area under the spectral curve of the apparent optical density (OD) in the 510 - 610 nm range, calculated as

$$EI_1 = 100 \times (OD_{560} + 1.5 \times [OD_{545} + OD_{575}] - 2.0 \times [OD_{510} + OD_{610}]), \qquad (8)$$

where this wavelength range was chosen to include the specific hemoglobin absorption peaks.

The second method was a simplified version based on comparison of the reflectance at a wavelength where hemoglobin absorptivity is high (560 nm) and at a wavelength where hemoglobin absorptivity is low (650 nm) [Zijlstra 2000]. The Erythema index was thus defined as

$$EI_2 = 100 \times (OD_{560} - OD_{650}). \qquad (9)$$

Both parameters correlated linearly with $\Delta AF$ and were used in the model.

Although it was not expected that erythema should be different as a function of skin color, it was expected that a combination of erythema index as calculated with the two suggested methods would yield a good estimate of melanin influence, because the simplified $EI_2$ method ignores the contribution of melanin absorption, while the first method ($EI_1$) should be independent of melanin absorption.

Furthermore, Feather et al. [Feather 1989] developed formulas that describe hemoglobin concentration and oxygenation as indices, based on measurements at isobestic points. These indices were included in the model as parameters

$$HI = 100 \times ([OD_{544} - OD_{527.5}] / 16.5 - [OD_{573} - OD_{544}] / 29) \qquad (10)$$

and

$$OI = (5100 / HI) \times ([OD_{573} - OD_{558.5}] / 14.5 - [OD_{558.5} - OD_{544}] / 14.5) + 42. \quad (11)$$

Bilirubin related parameters

Bilirubin has an absorption peak around 470 nm, which is within the emission range of the skin AF measurement, and has almost no absorption at 500 nm [Anderson 1981]. To assess the possible additional influence of bilirubin absorption, the ratio of the reflectance at 470 and 500 nm was included in the model as bilirubin index:

$$BI = R_{470} / R_{500}. \qquad (12)$$

Emission related parameters

It was expected that besides the reflectance spectra, also the emission spectra contained information that could be correlated to $\Delta$AF. Because absolute intensities are related to fluorophore content, only relative intensities can be used. Ratios of emission intensities at wavelength pairs 470 and 500 nm ($Em_1$), 470 and 570 nm ($Em_2$) as well as 600 and 650 nm ($Em_3$) were included as parameters. The ratio between mean emission in the 470-500 nm and 600 - 650 nm ranges was included as parameter $Em_4$.

*Univariate analyses*

In the dataset of 99 subjects, the parameters as described above were assessed for linear correlation with age-corrected AF, $\Delta$AF. Table 2 summarizes the univariate linear correlation coefficients (Pearson's $R^2$) that were found for correlations between $\Delta$AF and the various parameters. Because all parameters were designed or transformed as such, only linear correlations existed. Normality was assessed using the Kolmogorov-Smirnov test for each parameter. Significance values of normality (p) are also shown in Table 2. It should be noted that not all parameters had a normal distribution.

**Table 2.** Results from univariate linear correlations. For each parameter in the model, the square of Pearson's coefficient of correlation is presented ($R^2$). Normality is assessed using a one-sample Kolmogorov-Smirnov test. Values of p above 0.05 indicate a normal distribution.

| parameter | description | R2 | normality (P) |
|---|---|---|---|
| $MI_1$ | Ratio of reflectance at 390 and 360 nm | 0.701 | 0.27 |
| RedLnSlope | Slope of line through In reflectance in 630 - 675 nm | 0.681 | < 0.01 |
| $MI_3$ | Difference of OD at 620 and 675 nm | 0.676 | < 0.01 |
| $MI_2$ | Difference of OD at 650 and 675 nm | 0.651 | < 0.01 |
| $LnR_{500}$ | Natural logarithm of reflectance at 500 nm | 0.638 | < 0.01 |
| RedRefl | Mean reflectance in 620 - 675 nm range | 0.564 | < 0.01 |
| $dUV_{shape}$ | Deviation of UV reflectance from straight line | 0.541 | 0.65 |
| $EI_2$ | Difference of OD at 560 and 650 nm | 0.471 | 0.47 |
| InvRef1 | Inverse of reflectance in UV range | 0.452 | < 0.01 |
| **$EI_1$** | Area under curve of apparent OD spectrum in 510-610 nm range | 0.202 | 0.65 |
| HI | Hb absorption measured at isobestic points | 0.174 | 0.91 |
| $Em_4$ | Ratio of emission in 470 - 500 and 600 - 650 nm ranges | 0.115 | < 0.01 |
| Age | Subject age | 0.105 | 0.60 |
| $Em_3$ | Ratio of emission at 600 and 650 nm | 0.082 | < 0.01 |
| BI | Ratio of reflectance at 470 and 500 nm | 0.080 | 0.88 |
| $Em_1$ | Ratio of emission at 470 and 500 nm | 0.029 | < 0.01 |
| $Em_2$ | Ratio of emission at 470 and 570 nm | 0.004 | 0.20 |

(continued)

| parameter | description | R2 | normality (P) |
|---|---|---|---|
| OI | Oxygenation index based on ratio single/double absorption peak Hb | 0.001 | 0.04 |

OD is defined as - log $R_\lambda$.

### *Determination of the most preferred algorithm*

The formulated parameters as described above were used in a backward multiple linear regression analysis to find a model to describe $\Delta$AF. When a p = 0.05 threshold was used, four parameters contributed (dUV$_{shape}$ and the three parameters as listed in Table 3). The parameter with the lowest relative contribution, dUVshape, had a $\beta$ value less than half of that of the MI1 and RedLnSlope parameters. Herein, the standardized correlation coefficient $\beta$ represents the contribution of a specific parameter relative to the contribution of others. Adjusted $R^2$ was 0.814, not substantively different from the adjusted $R^2$ level of 0.804 with the three-parameter model, with a p < 0.01 threshold level, which is shown in Table 3. It is also possible to provide a reasonable prediction of other parameters are left out. If for instance the parameter subject age was not included, adjusted $R^2$ was 0.731. $\Delta$AF can thus to a substantive extent be predicted from the preferred linear combination of the parameters in Table 3, and the preferred new algorithm for calculating skin AF has been based on these parameters. It is noted that, dependent on available measurement instruments, it can in practice be preferable to use other ones of the formulated parameters. For instance by replacing RedLnSlope by a ratio of reflectances at for instance 630 nm and 675 nm (or ranges of for instance 5, 10 or 20 nm adjacent or around these values) or to replace MI$_1$ by the slope of the line through In in reflectance in 360-390 nm.

Collinearity was assessed as well. Although collinearity was found between some parameters, the significant parameters in the model are independent (tolerance above 0.01).

**Table 3.** Resulting parameters of the multiple regression analysis. The three-parameter model (p < 0.01 threshold) had an adjusted $R^2$ of 0.804.

| Parameter | β | p | Collinearity |
|---|---|---|---|
| *(constant)* | | 0.007 | |
| MI$_1$ | 0.406 | 0.000 | 0.226 |
| RedLnSlope | -0.463 | 0.000 | 0.228 |
| Age | -0.274 | 0.000 | 0.977 |

As can be seen from Table 2, a substantive predictive contribution can also be obtained from the logarithm of the reflectance at 500 nm. A similar predictive contribution can be expected from the reflectance at a wavelength or wavelength range in a range from 450 to 525 nm, because in that range the relative difference between the reflectance of a light skin and the reflectance of a dark skin (cf. Figure 2) is higher than in other wavelength ranges. It is also possible to use the relatively large difference between the reflectance of a light skin and the reflectance of a dark skin in the 450-525 nm wavelength range by using the ratio between the reflectance in a first wavelength or wavelength range in a range of 450-525 nm and the reflectance in a second wavelength or wavelength range in a range higher than 620 or 625 nm as a parameter in the correction of the measured AF for skin color.

A particular advantage of correcting in accordance with reflectances measured in the 450-525 nm and the over 620 nm wavelength ranges is that the disturbance due to differences in specular reflection are smaller than in the 350-400 nm wavelength range, where the level of diffuse reflection is relatively low.

In the apparatus according to the example shown in Fig. 8, the successive determination of AF and reflectances at the excitation wavelength(s), the 450-525 nm and the over 620 nm wavelength ranges can be achieved in a simple manner by sequentially switching the LEDs 102, 125 and 126 on, each next LED being switched on after the previous LED has been switched off, and reading out the detected light intensities from both detectors 120, 122 when the UV-A LED 102 is active and from either detector 120 or 122 or both when the 450-525 nm and the over 620 nm LEDs are active.

From the point of safety, it is preferred that inadvertent irradiation of UV-A radiation from the radiation source via the opening for irradiating the skin into the eyes of a subject or operator of the apparatus is avoided. Such irradiation with UV-A radiation is at least irritating and poses a health risk for the eyes in particular when occurring frequently. To avoid such inadvertent exposure of the eyes to UV-A radiation from the excitation radiation source 2, 102, the apparatuses are preferably arranged for preventing the excitation radiation source 2, 102 from being switched on unless the opening 8, 108 is covered by a skin surface to be analyzed. This is achieved by first measuring reflectance when the apparatus is switched on and only giving clearance for switching the UV-A light source 2, 102 on if no or only very little light is detected while the other light sources are inactive as well - the latter situation indicating that the opening 8, 108 is adequately covered.

A residual risk remains in the event the apparatus is left "ON" in a situation without ambient light or if a person puts the apparatus with the opening 8, 108 against an eye. To avoid such residual risks as well, the other light source(s) 19, 125, 126 may be activated prior to clearance for activation of the UV light source 2, 102, the clearance being given only and only for a limited time interval of for instance less than 1, 2 or 10 seconds, if the reflectance characteristics are within a range representative for a skin surface to be irradiated. It is observed that these safety features are of advantage also for apparatus that is not arranged for the correction of the measured skin AF for differences in skin color, but is of particular advantage in combination with means for analyzing reflectance characteristics for the correction for difference in skin color.

*Validation*

Using the preferred algorithm as obtained above, the corrected value of skin AF, $AF_{corr}$, was calculated using

$$AF_{corr} = AF_m + \alpha_1\, MI_1 + \alpha_2\, RedLnSlope + \alpha_3\, Age, \qquad (13)$$

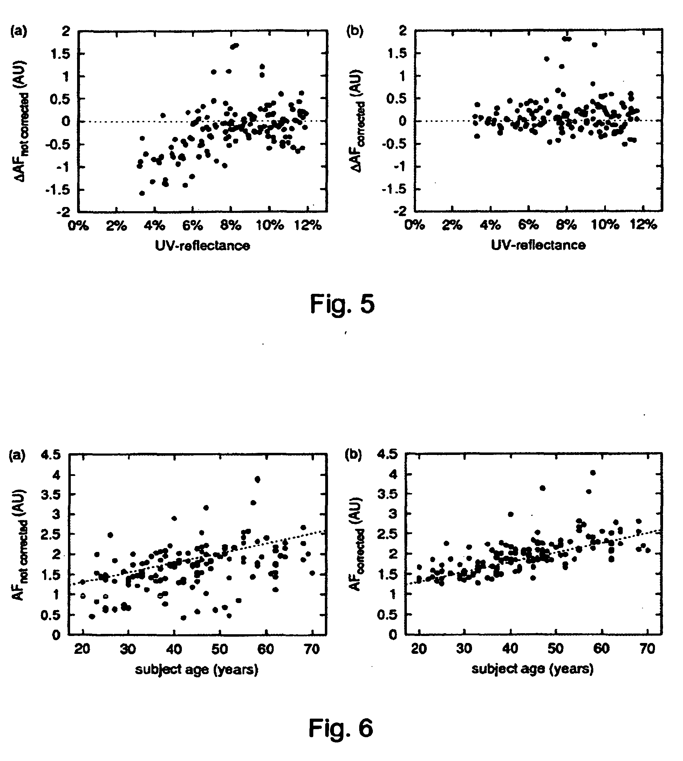

where $AF_m$ is the measured uncorrected skin AF, and $\alpha 1$ through $\alpha_3$ are multiplication constants that were derived using the multiple regression analysis. Skin AF ($AF_{corr}$) and age-adjusted skin AF ($\Delta AF_{corr}$) were calculated for each individual in the validation-group. $\Delta AF_{corr}$ was calculated using Eq. (1), using $AF_{corr}$ instead of $AF_m$. This group consisted of 27 subjects from the Afro-Caribbean cohort, 87 subjects from the South Chinese cohort and 27 from the cohort of subjects of various origin. Age-adjusted skin AF is shown as a function of UV-reflectance values in Figure 5, also comparing the new algorithm (b) and the old method for calculating skin AF (a). With the new algorithm, the mean standard deviation of $\Delta AF_{corr}$ as percentage of the skin AF is 14.8%.

Figure 6 shows the AF values as a function of subject age, as calculated with the new algorithm (b) as well as without correction for skin color (a). Values are compared with the standard reference line as obtained from Caucasian subjects [Koetsier 2010].

**Discussion**

The current invention provides a new calculation algorithm for skin AF, that enables reliable determination of increased skin AF in subjects having different skin colors. For this algorithm, parameters were formulated and applied to reflectance spectra as measured on the skin. Selected sets of a small number of the formulated parameters correlate with the originally observed decrease in skin AF values of subjects with a dark skin color very well.

The present invention allows correcting measured AF values for difference in skin color by using spectra that are measured individually in the UV-A and visible range (eg. 350 - 675 nm) and therefore using the same sensors that are used for measuring reflectance and fluorescence to obtain the ration between these values as well as the same radiation sources that are used for generating the excitation radiation and the emission for measuring reflectance at the wavelength ramge of the fluorescence. For formulating this correction, the main characteristics of only the strongest contributing absorbers, melanin, hemoglobin and bilirubin, have initially been used as a basis for formulating significant spectral properties that may predict the lower skin AF values in subjects with a dark skin color.

The preferred model, using subject age and two parameters from the reflectance spectrum, did account for over 80% of the relative change in skin AF values in the set of subjects to which the preferred model was applied. The new calculation algorithm, based on this model, yields skin AF values that are almost independent of skin color, even without knowing the exact composition of chromophores, fluorophores and scattering particles in the skin.

If a 0.05 threshold was used, the additional $dUV_{shape}$ parameter would be included, which had a $\beta$ value of less than half of that of the other two parameters, $MI_1$ and RedLnSlope. Adjusted $R^2$ was not better than for the preferred model with only two spectral parameters. Therefore, in this study, the low threshold of 0.01 was chosen for excluding parameters from the model.

In the current study, only age and the $MI_1$ and RedLnSlope parameters of Table 1 were necessary to describe the influence of skin color on skin AF. All other parameters, including the parameters from the emission spectra, could be discarded from the model. A bilirubin related parameter appeared promising as well because we initially assumed that small changes might also influence the measured skin AF, but was found to be of little influence. Nevertheless, a significant influence of this parameter on skin AF may be present in conditions such as jaundice. Similarly, the present results can not exclude that strong erythema may also influence skin AF.

Subject age is an important predictor for skin AF values. Therefore, an age-corrected value, based on reference values of skin AF in Caucasian subjects [Koetsier 2010], was used in the model. However, the age-dependence of skin AF may be different depending on racial or cultural differences, e.g. dietary variations or smoking habits. By applying the same relation of corrected skin AF and age to all subjects, equal reference values can be used, allowing the detection of increased skin AF independent on skin color. Our results show that corrected AF has the same increase with subject age for the entire group of subjects from various descent.

Figure 5 shows some subjects that have higher skin AF values than the other subjects of the same age, even after correction (ΔAF value above 1). We assume that these subjects may have developed an increased cardiovascular risk, without immediate clinical symptoms. It should be noted that in the cohort that was used for developing the model, no increased values of skin AF were observed (not shown).

The inclusion of subject age in the model may seem unnecessary at first, because the model was designed to predict ΔAF, which reflects a value independent of age. However, it was assumed that age could have an effect on other parameters. Although age did not correlate to any of the parameters, it turned out to be a significant predictor in the model. If age is left out from the model, adjusted $R^2$ decreases to 0.731.

Although we did not yet attempt to physically explain our observations, the current study suggests that for the purpose of assessing skin AGEs, the influence of skin color on the AF measurements may be sufficiently described using age and the $MI_1$ and RedLnSlope parameters, i.e. the ratio of two reflectance values in the 360 - 390 nm range and the slope of the reflectance in the 620 - 675 nm range or corresponding parameters describing the relationship between the reflectances at wavelengths or wavelength ranges at the opposite ends of the excitation wavelength range and the relationship between the reflectances at different wavelengths or wavelength ranges in a range over 620 nm or over 625 nm, for instance up to 675 or up to 880 or 900 nm up to which level the slope of the reflectance curve differs significantly between light and dark coloured skin. In the presently preferred model, this resulted in a mean standard deviation of 14.8% of the AF values, which is even lower than the 20% that was observed in a Caucasian group from an earlier study [Koetsier 2010]. Therefore, the present invention provides a technique to recognize increased values of skin AF independent of skin color, with a reliability that is at least similar to the reliability previously achievable for subjects of light skin color only.It is noted that it is possible to apply combinations of measured AF values and corrected AF values. For example, it is possible to use an AF value of the form:

$$AF = x\, AF_m + (1 - x)\, AF_{corr} ;$$

wherein $AF_m$ is a measured, i.e. uncorrected, AF value, $AF_{corr}$ is a corrected AF value and x, whose value is between zero and one, is a function of the UV-reflectance of a subject, such that e.g. x = 1 when the UV-reflectance of a subject exceeds (say) 10% and e.g. x = 0 when the UV-reflectance of a subject is less than (say) 3%. Then, if x = 1, AF = $AF_m$, and, if x = 0, AF = $AF_{corr}$. In this case, no correction is applied for subjects with a high UV-reflectance, while the full correction is applied for subjects with a very low UV-reflectance, while in said example a correction is proportionally applied to subjects having an intermediate UV-reflectance.

It is further noted that the proposed solution for correcting measured AF is particularly suitable for implementation in a simple manner, because no or very few additional hardware is required. If the instrument is equipped with a spectrometer, a white or other light source having a sufficiently broad emission spectrum is sufficient to allow analysis of the reflection spectrum to obtain the proposed parameters. Alternatively, the apparatus only needs to be arranged for selectively emitting and/or detecting light of specific wavelengths or wavelengths ranges only, such as the apparatus according to the example shown in Fig. 8.

This makes the measurement of skin AF for the non-invasive assessment of increased levels of skin AGEs more generally applicable.

The above description is partly based on a publication by M. Koetsier et al., "Skin color independent assessment of aging using skin autofluorescence.", accepted by Optics Express (© 2010 Optical Society of America, Inc.).

### Cited Literature

[Alaluf 2002] S. Alaluf, D. Atkins, K. Barrett, M. Blount, N. Carter, and A. Heath, "Ethnic variation in melanin content and composition in photoexposed and photoprotected human skin.," Pigment cell research / sponsored by the European Society for Pigment Cell Research and the International Pigment Cell Society, vol. 15, 2002, pp. 112-8.

[Anderson 1981] R.R. Anderson and J.A. Parrish, "The optics of human skin.," J Invest Dermatol, vol. 77, 1981, pp. 13-19.

[Bachmann 2006] L. Bachmann, D. Zezell, A. Ribeiro, L. Gomes, and A. Ito, "Fluorescence spectroscopy of biological tissues-A review," Applied Spectroscopy Reviews, vol. 41, 2006, p. 575-590.

[Barsh 2003] G.S. Barsh, "What controls variation in human skin color?," PLoS Biol, vol. 1, 2003, p. E27.

[Chen 2007] R. Chen, Z. Huang, H. Lui, I. Hamzavi, D.I. McLean, S. Xie, and H. Zeng, "Monte Carlo simulation of cutaneous reflectance and fluorescence measurements--the effect of melanin contents and localization.," J Photochem Photobiol B, vol. 86, 2007, pp. 219-226.

[Coremans 1997] J.M. Coremans, C. Ince, H.A. Bruining, and G.J. Puppels, "(Semi-)quantitative analysis of reduced nicotinamide adenine dinucleotide fluorescence images of blood-perfused rat heart.," Biophys J, vol. 72, 1997, pp. 1849-1860.

[Dawson 1980] J.B. Dawson, D.J. Barker, D.J. Ellis, E. Grassam, J.A. Cotterill, G.W. Fisher, and J.W. Feather, "A theoretical and experimental study of light absorption and scattering by in vivo skin.," Phys Med Biol, vol. 25, 1980, pp. 695-709.

[den Hollander 2007] N.C. den Hollander, D.J. Mulder, R. Graaff, S.R. Thorpe, J.W. Baynes, G.P. Smit, and A.J. Smit, "Advanced glycation end products and the absence of premature atherosclerosis in glycogen storage disease Ia.," J Inherit Metab Dis, vol. 30, 2007, pp. 916-923.

[Feather 1989] J.W. Feather, M. Hajizadeh-Saffar, G. Leslie, and J.B. Dawson, "A portable scanning reflectance spectrophotometer using visible wavelengths for the rapid measurement of skin pigments.," Phys Med Biol, vol. 34, 1989, pp. 807-820.

[Katika 2006] K.M. Katika and L. Pilon, "Steady-state directional diffuse reflectance and fluorescence of human skin.," Appl Opt, vol. 45, 2006, pp. 4174-4183.

[Koetsier 2009] M. Koetsier, H.L. Lutgers, A.J. Smit, T.P. Links, R.D. Vries, R.O. Gans, G. Rakhorst, and R. Graaff, "Skin autofluorescence for the risk assessment of chronic complications in diabetes: a broad excitation range is sufficient.," Opt Express, vol. 17, 2009, pp. 509-519.

[Koetsier 2010] M. Koetsier, H.L. Lutgers, C. de Jonge, T.P. Links, A.J. Smit, and R. Graaff, "Reference values of skin autofluorescence.," Diabetes Technol Ther, vol. 12, 2010, pp. 399-403.

[Kollias 1985] N. Kollias and A. Baqer, "Spectroscopic characteristics of human melanin in vivo.," J Invest Dermatol, vol. 85, 1985, pp. 38-42.

[Kollias 1987] N. Kollias and A.H. Baqer, "Absorption mechanisms of human melanin in the visible, 400-720 nm.," J Invest Dermatol, vol. 89, 1987, pp. 384-388.

[Kollias 2002] N. Kollias, G. Zonios, and G.N. Stamatas, "Fluorescence spectroscopy of skin.," Vib Spectrosc, vol. 28, 2002, pp. 17-23.

[Lutgers 2009] H.L. Lutgers, E.G. Gerrits, R. Graaff, T.P. Links, W.J. Sluiter, R.O. Gans, H.J. Bilo, and A.J. Smit, "Skin autofluorescence provides additional information to the UK Prospective Diabetes Study (UKPDS) risk score for the estimation of cardiovascular prognosis in type 2 diabetes mellitus.," Diabetologia, vol. 52, 2009, pp. 789-797.

[Magnain 2007] C. Magnain, M. Elias, and J. Frigerio, "Skin color modeling using the radiative transfer equation solved by the auxiliary function method.," Journal of the Optical Society of America. A, Optics, image science, and vision, vol. 24, 2007, pp. 2196-205.

[Matsumoto 2007] T. Matsumoto, T. Tsurumoto, H. Baba, M. Osaki, H. Enomoto, A. Yonekura, H. Shindo, and T. Miyata, "Measurement of advanced glycation endproducts in skin of patients with rheumatoid arthritis, osteoarthritis, and dialysis-related spondyloarthropathy using non-invasive methods.," Rheumatol Int, vol. 28, 2007, pp. 157-160.

[Meerwaldt 2004] R. Meerwaldt, R. Graaff, P.H. Oomen, T.P. Links, J.J. Jager, N.L. Alderson, S.R. Thorpe, J.W. Baynes, R.O. Gans, and A.J. Smit, "Simple non-invasive assessment of advanced glycation endproduct accumulation.," Diabetologia, vol. 47, 2004, pp. 1324-1330.

[Meerwaldt 2005] R. Meerwaldt, J.W. Hartog, R. Graaff, R.J. Huisman, T.P. Links, N.C. den Hollander, S.R. Thorpe, J.W. Baynes, G. Navis, R.O. Gans, and A.J. Smit, "Skin autofluorescence, a measure of cumulative metabolic stress and advanced glycation end products, predicts mortality in hemodialysis patients.," J Am Soc Nephrol, vol. 16, 2005, pp. 3687-3693.

[Monami 2008] M. Monami, C. Lamanna, F. Gori, F. Bartalucci, N. Marchionni, and E. Mannucci, "Skin autofluorescence in type 2 diabetes: beyond blood glucose.," Diabetes Res Clin Pract, vol. 79, 2008, pp. 56-60.

[Mulder 2008] D.J. Mulder, P.L. van Haelst, S. Gross, K. de Leeuw, J. Bijzet, R. Graaff, R.O. Gans, F. Zijlstra, and A.J. Smit, "Skin autofluorescence is elevated in patients with stable coronary artery disease and is associated with serum levels of neopterin and the soluble receptor for advanced glycation end products.," Atherosclerosis, vol. 197, 2008, pp. 217-223.

[Mulder 2006] D.J. Mulder, T.V. Water, H.L. Lutgers, R. Graaff, R.O. Gans, F. Zijlstra, and A.J. Smit, "Skin autofluorescence, a novel marker for glycemic and oxidative stress-derived advanced glycation endproducts: an overview of current clinical studies, evidence, and limitations.," Diabetes Technol Ther, vol. 8, 2006, pp. 523-535.

[Na 2001] R. Na, I.M. Stender, M. Henriksen, and H.C. Wulf, "Autofluorescence of human skin is age-related after correction for skin pigmentation and redness.," J Invest Dermatol, vol. 116, 2001, pp. 536-540.

[Nielsen 2008] K.P. Nielsen, L. Zhao, G.A. Ryzhikov, M.S. Biryulina, E.R. Sommersten, J.J. Stamnes, K. Stamnes, and J. Moan, "Retrieval of the physiological state of human skin from UV-Vis reflectance spectra - a feasibility study.," J Photochem Photobiol B, vol. 93, 2008, pp. 23-31.

[Nishidate 2004] I. Nishidate, Y. Aizu, and H. Mishina, "Estimation of melanin and hemoglobin in skin tissue using multiple regression analysis aided by Monte Carlo simulation.," Journal of biomedical optics, vol. 9, 2004, pp. 700-10.

[Sandby-Møller 2003] J. Sandby-Møller, T. Poulsen, and H.C. Wulf, "Influence of epidermal thickness, pigmentation and redness on skin autofluorescence.," Photochem Photobiol, vol. 77, 2003, pp. 616-620.

[Sinichkin 2002] Y.P. Sinichkin, N. Kollias, G.I. Zonios, S.R. Utz, and V.V. Tuchin, "Reflectance and fluorescence spectroscopy of human skin in vivo," Handbook of optical biomedical diagnostics, V.V. Tuchin, Bellingham, WA, USA: SPIE Press, 2002, pp. 725-785.

[Stamatas 2006] G.N. Stamatas, R.B. Estanislao, M. Suero, Z.S. Rivera, J. Li, A. Khaiat, and N. Kollias, "Facial skin fluorescence as a marker of the skin's response to chronic environmental insults and its dependence on age.," Br J Dermatol, vol. 154, 2006, pp. 125-132.

[Ueno 2008] H. Ueno, H. Koyama, S. Tanaka, S. Fukumoto, K. Shinohara, T. Shoji, M. Emoto, H. Tahara, R. Kakiya, T. Tabata, T. Miyata, and Y. Nishizawa, "Skin autofluorescence, a marker for advanced glycation end product accumulation, is associated with arterial stiffness in patients with end-stage renal disease.," Metabolism, vol. 57, 2008, pp. 1452-1457.

[Zijlstra 2000] W.G. Zijlstra, A. Buursma, and O.W. van Assendelft, Visible and near infrared absorption spectra of human and animal haemoglobin, Zeist, The Netherlands: VSP BV, 2000.

[Zonios 2006] G. Zonios and A. Dimou, "Modeling diffuse reflectance from semi-infinite turbid media: application to the study of skin optical properties," Opt Express, vol. 14, 2006, pp. 8661-8674.

[Zonios 2008a] G. Zonios, A. Dimou, I. Bassukas, D. Galaris, A. Tsolakidis and E. Kaxiras, "Melanin absorption spectroscopy: new method for noninvasive skin investigation and melanoma detection," J Biomed Opt, vol. 13, 2008, 14017.

[Zonios 2008b] G. Zonios and A. Dimou, "Melanin optical properties provide evidence for chemical and structural disorder in vivo.," Opt Express, vol. 16, 2008, pp. 8263-8268.

**Claims**

1. A method for determining an autofluorescence value of skin tissue of a subject, comprising the steps of:

   - irradiating material of said skin tissue with electromagnetic excitation radiation of a wavelength or wavelength range within the excitation range of wavelengths of 300-420 nm;
   - measuring an amount of electromagnetic, fluorescent radiation emitted by said material in response to said irradiation over a range of wavelengths of 420-600 nm; and
   - generating, based upon said measured amount of fluorescent radiation, a measured autofluorescence value for the concerning subject;
   - measuring a first measured reflectance at a first wavelength or wavelength range and a second measured reflectance at a second wavelength or wavelength range different from said first wavelength or wavelength range, wherein said first and second wavelengths or wavelength ranges are both within the excitation range of wavelengths with which the skin tissue is irradiated for excitation, or both within the range of wavelengths in which the emitted fluorescent radiation is measured, or both in a range of wavelengths higher than 620 nm, or wherein said first wavelength or wavelength range is in a range of wavelengths of 450-525 nm and said second wavelength or wavelength range is in a range of wavelengths higher than 620 nm; and
   - generating a corrected autofluorescence value by correcting the measured autofluorescence in accordance with a relationship between said first and second measured reflectances in such manner that the dependency of the corrected autofluorescence value upon different UV-skin tissue reflectances, that different respective subjects may have, is minimized or at least diminished

2. A method according to claim 1, wherein the first wavelength or wavelength range is in a range of 360-365 nm and wherein the second wavelength or wavelength range is in a range of 390-395 nm.

3. A method according to claim 1, wherein the first wavelength or wavelength range is in a range of 620-650 nm and wherein the second wavelength or wavelength range is in a range higher than 675 nm.

4. A method according to claim 1, said first wavelength or wavelength range is in said range of 450-525 nm, wherein said second wavelength or wavelength range is in a range higher than 620 or 625 nm.

5. A method according to claim 1, said first wavelength or wavelength range being in said range of wavelengths of 450-525 nm and said second wavelength or wavelength range being in said range of wavelengths higher than 620 nm, or according to claim 3 or 4, wherein the second wavelength or wavelength range is below 900 nm.

6. A method according to any of the preceding claims, wherein the correction is for a ratio between reflectance at the first wavelength or wavelength range and at the second wavelength or wavelength range.

7. A method according to any of the preceding claims, wherein the relationship between the first measured reflectance at the first wavelength or wavelength range and the second measured reflectance at the second wavelength or wavelength range is obtained by determining a slope of a graph from a logarithm of the first measured reflectance at the first wavelength or wavelength range to a logarithm of the second measured reflectance at the second wavelength or wavelength range.

8. A method according to claim 7, wherein the graph of which a slope is determined represents, on a logarithmic scale, a reflection spectrum obtained from the subject at wavelengths above 620 nm.

9. A method according to claim 1, wherein the corrected autofluorescence value is obtained by the formula:

$$\mathrm{AF_{corr} = AF_m + \alpha_1\, MI_1 + \alpha_2\, RedLnSlope + \alpha_3\, Age}$$

in which:

- $AF_{corr}$ is the corrected autofluorescence value;
- $AF_m$ is the measured, i.e. uncorrected, autofluorescence value;
- $MI_1$ is a ratio of reflectance values against said skin tissue at two different wavelengths in a range 300 nm - 420 nm of said excitation radiation, or a slope in a logarithmic scale of a reflection spectrum in said range 300 nm - 420 nm;
- RedLnSlope is a slope in a logarithmic scale of a reflection spectrum at wavelengths above 620 nm, or a ratio between reflectance values at wavelengths above 620 nm;
- Age is the age of the concerning subject; and
- $\alpha_1$, $\alpha_2$ and $\alpha_3$ are coefficients determined e.g. by regression analysis on a dataset of subjects.

10. A method according to any of the preceding claims, wherein the corrected autofluorescence value is obtained by correcting the measured autofluorescence in accordance with deviation of reflectance over the UV wavelength range (300 - 420 nm) from a straight line.

11. A method according to any of the preceding claims, wherein an estimate of an Advanced Glycation Endproducts (AGEs) content accumulated in said (skin) tissue of the concerning subject is made based upon the corrected autofluorescence value.

12. A method according to claim 11, wherein an estimate of Advanced Glycation Endproducts (AGEs) content or subject's health risk is made based on a combination of the measured autofluorescence value and the corrected autofluorescence value.

13. An apparatus for determining an autofluorescence value of skin tissue of a subject, comprising:

a pick-up unit comprising radiation means, for in vivo and noninvasively irradiating intact skin tissue behind a particular irradiation window with electromagnetic excitation radiation of a wavelength or wavelength range within the excitation range of wavelengths of 300-420 nm,
detection means for measuring electromagnetic fluorescent radiation coming from said skin tissue over a range of wavelengths of 420-600 nm ; and
generation means for generating a measured autofluorescence value for said tissue in agreement with said measured amount of fluorescent radiation originating from said tissue;
said radiation detection and generation means being further arranged for obtaining a first measured reflectance at a first wavelength or wavelength range and a second measured reflectance at a second wavelength or wavelength range different from said first wavelength or wavelength range said first and second wavelengths or wavelength ranges are both within the excitation range of wavelengths with which the skin tissue is irradiated for excitation, both within the range of wavelengths in which the emitted fluorescent radiation is measured, or both in a range of wavelengths higher than 620 nm, or said first wavelength or wavelength range is in a range of wavelengths of 450-525 nm and said second wavelength or wavelength range is in a range of wavelengths higher than 620 nm; and

said generation means being arranged for correcting the measured autofluorescence in accordance with a relationship between said first and second measured reflectances in such a manner that the dependency of the corrected autofluorescence value upon different UV-skin tissue reflectances that different respective subjects may have, is minimized or at least diminished.

14. Apparatus according to claim 13, wherein the radiation means are arranged for selectively emitting radiation in at least two mutually distinct wavelengths or wavelength ranges in a range of wavelengths higher than 420 nm; and wherein the detection means are arranged for selectively detecting reflection in at least two mutually distinct wavelengths or wavelength ranges in the range of wavelengths higher than 420 nm.

15. An apparatus according to claim 13 or 14, arranged for preventing activation of the radiation means to emit the electromagnetic excitation radiation unless no radiation is detected by the detection means.

16. An apparatus according to claim 15, wherein the radiation means are further adapted for irradiating skin tissue behind the irradiation window with electromagnetic radiation in at least one range of wavelengths higher than 420 nm, and

wherein said apparatus is arranged for preventing activation of the radiation means to emit the electromagnetic excitation radiation unless a reflectance meeting a reference characteristic for a skin to be irradiated is detected by the detection means when the skin is irradiated by the radiation means with said electromagnetic radiation in at least the range of wavelengths higher than 420 nm.


**Patentansprüche**

1. Verfahren zur Bestimmung eines Autofluoreszenzwertes von Hautgewebe eines Patienten, umfassend folgende Schritte:

- Bestrahlung von Material des Hautgewebes mit elektromagnetischer Erregungsstrahlung mit einer Wellenlänge oder einem Wellenlängenbereich innerhalb des Erregungsbereichs von Wellenlängen von 300-420 nm;
- Messung einer Menge von elektromagnetischer fluoreszierender Strahlung, emittiert von dem Material in Reaktion auf die Bestrahlung über einen Bereich von Wellenlängen von 420-600 nm; und
- Erzeugung, basierend auf der gemessenen Menge der fluoreszierenden Strahlung, eines gemessenen Autofluoreszenzwertes für den betreffenden Patienten;
- Messung einer ersten gemessenen Reflektanz bei einer ersten Wellenlänge oder einem ersten Wellenlängenbereich und einer zweiten gemessenen Reflektanz bei einer zweiten Wellenlänge oder einem zweiten Wellenlängenbereich, verschieden von der ersten Wellenlänge oder dem ersten Wellenlängenbereich, wobei
- die ersten und zweiten Wellenlängen oder Wellenlängenbereiche beide innerhalb des Erregungsbereichs von Wellenlängen sind, mit denen das Hautgewebe zur Erregung bestrahlt wird, oder beide innerhalb des Bereichs von Wellenlängen sind, in dem die emittierte fluoreszierende Strahlung gemessen wird, oder beide in einem Bereich von Wellenlängen über 620 nm sind, oder wobei die erste Wellenlänge oder der erste Wellenlängenbereich in einem Bereich von Wellenlängen von 450-525 nm ist und die zweite Wellenlänge oder der zweite Wellenlängenbereich in einem Bereich von Wellenlängen über 620 nm ist; und
- Erzeugung eines korrigierten Autofluoreszenzwertes durch Korrektur der gemessenen Autofluoreszenz in Übereinstimmung mit einer Beziehung zwischen den ersten und zweiten gemessenen Reflektanzen in einer solchen Weise, dass die Abhängigkeit des korrigierten Autofluoreszenzwertes von verschiedenen UV-Hautgewebereflektanzen, die verschiedene Patienten jeweils aufweisen können, minimiert oder zumindest reduziert wird.

2. Verfahren nach Anspruch 1, wobei die erste Wellenlänge oder der erste Wellenlängenbereich in einem Bereich von 360-365 nm ist und wobei die zweite Wellenlänge oder der zweite Wellenlängenbereich in einem Bereich von 390-395 nm ist.

3. Verfahren nach Anspruch 1, wobei die erste Wellenlänge oder der erste Wellenlängenbereich in einem Bereich von 620-650 nm ist und wobei die zweite Wellenlänge oder der zweite Wellenlängenbereich in einem Bereich über 675 nm ist.

4. Verfahren nach Anspruch 1, wobei die erste Wellenlänge oder der erste Wellenlängenbereich in dem Bereich von 450-525 nm ist, wobei die zweite Wellenlänge oder der zweite Wellenlängenbereich in einem Bereich über 620 oder 625 nm ist.

5. Verfahren nach Anspruch 1, wobei die erste Wellenlänge oder der erste Wellenlängenbereich in dem Bereich von Wellenlängen von 450-525 nm ist und die zweite Wellenlänge oder der zweite Wellenlängenbereich in dem Bereich von Wellenlängen über 620 nm ist, oder nach Anspruch 3 oder 4, wobei die zweite Wellenlänge oder der zweite Wellenlängenbereich unter 900 nm ist.

6. Verfahren nach einem der vorhergehenden Ansprüche, wobei die Korrektur für ein Verhältnis zwischen Reflektanz bei der ersten Wellenlänge oder dem ersten Wellenlängenbereich und bei der zweiten Wellenlänge oder dem zweiten Wellenlängenbereich ist.

7. Verfahren nach einem der vorhergehenden Ansprüche, wobei die Beziehung zwischen der ersten gemessenen Reflektanz bei der ersten Wellenlänge oder dem ersten Wellenlängenbereich und der zweiten gemessenen Reflektanz bei der zweiten Wellenlänge oder dem zweiten Wellenlängenbereich durch Bestimmung einer Neigung einer grafischen Darstellung von einem Logarithmus der ersten gemessenen Reflektanz bei der ersten Wellenlänge oder dem ersten Wellenlängenbereich zu einem Logarithmus der zweiten gemessenen Reflektanz bei der zweiten Wellenlänge oder dem zweiten Wellenlängenbereich erhalten wird.

8. Verfahren nach Anspruch 7, wobei die grafische Darstellung, anhand derer eine Neigung bestimmt wird, auf einer logarithmischen Skala ein Reflexionsspektrum darstellt, das von dem Patienten bei Wellenlängen über 620 nm erhalten wurde.

9. Verfahren nach Anspruch 1, wobei der korrigierte Autofluoreszenzwert erhalten wird mittels der Formel:

$$\mathrm{AF_{corr}} = \mathrm{AF_m} + \alpha_1\,\mathrm{MI_1} + \alpha_2\,\mathrm{RedLnSlope} + \alpha_3\,\mathrm{Alter}$$

in der:

- $\mathrm{AF_{corr}}$ der korrigierte Autofluoreszenzwert ist;
- $\mathrm{AF_m}$ der gemessene, d. h. nicht korrigierte, Autofluoreszenzwert ist;
- $\mathrm{MI_1}$ ein Verhältnis von Reflektanzwerten gegen das Hautgewebe bei zwei verschiedenen Wellenlängen in einem Bereich von 300 nm - 420 nm der Erregungsstrahlung oder eine Neigung in einer logarithmischen Skala eines Reflexionsspektrum in dem Bereich 30 nm - 420 nm ist;
- RedLnSlope eine Neigung in einer logarithmischen Skala eines Reflexionsspektrums bei Wellenlängen über 620 nm oder ein Verhältnis zwischen Reflektanzwerten bei Wellenlängen über 620 nm ist;
- Alter das Alter des betreffenden Patienten ist; und
- $\alpha_1$, $\alpha_2$ und $\alpha_3$ Koeffizienten sind, bestimmt z. B. durch Regressionsanalyse an einem Datensatz von Patienten.

10. Verfahren nach einem der vorhergehenden Ansprüche, wobei der korrigierte Autofluoreszenzwert durch Korrigieren der gemessenen Autofluoreszenz in Übereinstimmung mit der Abweichung von Reflektanz über den UV-Wellenlängenbereich (300 - 420 nm) von einer Geraden erhalten wird.

11. Verfahren nach einem der vorhergehenden Ansprüche, wobei eine Schätzung des Inhalts eines AGEs (engl. Advanced Glycation Endproducts), der sich in dem (Haut)-Gewebe des betreffenden Patienten angesammelt hat, basierend auf dem korrigierten Autofluoreszenzwert gemacht wird.

12. Verfahren nach Anspruch 11, wobei eine Schätzung des Inhalts von AGEs (engl. Advanced Glycation Endproducts) oder des Gesundheitsrisikos des Patienten basierend auf einer Kombination des gemessenen Autofluoreszenzwertes und des korrigierten Autofluoreszenzwertes gemacht wird.

13. Vorrichtung zur Bestimmung eines Autofluoreszenzwertes von Hautgewebe eines Patienten, umfassend:

eine Aufnahmeeinheit, umfassend Strahlungsmittel für in vivo und nicht-invasive Bestrahlung von intaktem Hautgewebe hinter einem bestimmten Bestrahlungsfenster mit elektromagnetischer Erregungsstrahlung mit einer Wellenlänge oder einem Wellenlängenbereich innerhalb des Erregungsbereichs von Wellenlängen von 300-420 nm;

Detektionsmittel zum Messen elektromagnetischer fluoreszierender Strahlung, die von dem Hautgewebe kommt, über einen Bereich von Wellenlängen von 420-600 nm; und

Erzeugungsmittel zum Erzeugen eines gemessenen Autofluoreszenzwertes für das Gewebe in Übereinstimmung mit der gemessenen Menge an fluoreszierender Strahlung, ausgehend von dem Gewebe;

wobei die Mittel zur Strahlungsdetektion und Erzeugung ferner geeignet sind, um eine erste gemessene Reflektanz bei einer ersten Wellenlänge oder einem ersten Wellenlängenbereich und eine zweite gemessene Reflektanz bei einer zweiten Wellenlänge oder einem zweiten Wellenlängenbereich, verschieden von der ersten Wellenlänge oder dem ersten Wellenlängenbereich, zu erhalten;

die ersten und zweiten Wellenlängen oder Wellenlängenbereiche beide innerhalb des Erregungsbereichs von Wellenlängen sind, mit denen das Hautgewebe zur Erregung bestrahlt wird, beide innerhalb des Bereichs von Wellenlängen sind, in dem die emittierte fluoreszierende Strahlung gemessen wird, oder beide in einem Bereich von Wellenlängen über 620 nm sind, oder die erste Wellenlänge oder der erste Wellenlängenbereich in einem Bereich von Wellenlängen von 450-525 nm ist und die zweite Wellenlänge oder der zweite Wellenlängenbereich in einem Bereich von Wellenlängen über 620 nm ist; und

die Generierungsmittel geeignet sind, um die gemessene Autofluoreszenz in Übereinstimmung mit einer Beziehung zwischen den ersten und zweiten gemessenen Reflektanzen so zu korrigieren, dass die Abhängigkeit des korrigierten Autofluoreszenzwertes von verschiedenen UV-Hautgewebereflektanzen, die verschiedene Patienten jeweils haben können, minimiert oder zumindest reduziert wird.

14. Vorrichtung nach Anspruch 13, wobei die Strahlungsmittel geeignet sind, um Strahlung in mindestens zwei voneinander verschiedenen Wellenlängen oder Wellenlängenbereichen in einem Bereich von Wellenlängen über 420 nm selektiv zu emittieren; und wobei die Detektionsmittel geeignet sind, um Reflexion in mindestens zwei voneinander verschiedenen Wellenlängen oder Wellenlängenbereichen im Bereich von Wellenlängen über 420 nm selektiv zu detektieren.

15. Vorrichtung nach Anspruch 13 oder 14, geeignet um die Aktivierung der Strahlungsmittel, um die elektromagnetische Erregungsstrahlung zu emittieren, verhindert wird, es sei denn, die Detektionsmittel detektieren keine Strahlung.

16. Vorrichtung nach Anspruch 15, wobei die Strahlungsmittel ferner geeignet sind, Hautgewebe hinter dem Bestrahlungsfenster mit elektromagnetischer Strahlung in mindestens einem Bereich von Wellenlängen über 420 nm zu bestrahlen, und

wobei die Vorrichtung geeignet ist, die Aktivierung der Strahlungsmittel, um die elektromagnetische Erregungsstrahlung zu emittieren, zu verhindern, es sei denn, eine Reflektanz, die einem Referenzmerkmal einer zu bestrahlenden Haut entspricht, wird von den Detektionsmitteln detektiert, wenn die Haut von den Strahlungsmitteln mit der elektromagnetischen Strahlung in mindestens dem Bereich von Wellenlängen über 420 nm bestrahlt wird.

## Revendications

1. Procédé destiné à déterminer la valeur d'autofluorescence d'un tissu cutané d'un sujet, comprenant les étapes consistant à :

- irradier un matériau dudit tissu cutané avec un rayonnement d'excitation électromagnétique qui présente une longueur d'onde ou une plage de longueurs d'onde qui se situe à l'intérieur de la plage de longueurs d'onde d'excitation comprise entre 300 nm et 420 nm ;
- mesurer une quantité de rayonnement fluorescent électromagnétique émis par ledit matériau en réponse à ladite irradiation dans une plage de longueurs d'onde comprise entre 420 nm et 600 nm ; et
- générer, sur la base de ladite quantité mesurée de rayonnement fluorescent, une valeur d'autofluorescence mesurée du sujet en question ;
- mesurer un premier facteur de réflexion mesuré à une première longueur d'onde ou dans une première plage de longueurs d'onde, et un second facteur de réflexion mesuré à une seconde longueur d'onde ou dans une seconde plage de longueurs d'onde, différente de la ladite première longueur d'onde ou de ladite première plage de longueurs d'onde ;
dans lequel lesdites premières et secondes longueurs d'onde ou plages de longueurs d'onde, se situent toutes

EP 2 582 293 B1

deux à l'intérieur de la plage de longueurs d'onde d'excitation avec lesquelles le tissu cutané est irradié pour être excité, ou se situent toutes deux à l'intérieur de la plage de longueurs d'onde dans laquelle le rayonnement fluorescent émis est mesuré, ou se situent toutes deux dans une plage de longueurs d'onde supérieures à 620 nm, ou dans lequel ladite première longueur d'onde ou ladite première plage de longueurs d'onde se situe dans une plage de longueurs d'onde comprise entre 450 nm et 525 nm, et ladite seconde longueur d'onde ou ladite seconde plage de longueurs d'onde se situe dans une plage de longueurs d'onde supérieure à 620 nm ; et
- générer une valeur d'autofluorescence corrigée en corrigeant l'autofluorescence mesurée selon une relation entre lesdits premier et second facteurs de réflexion mesurés de telle façon que la dépendance de la valeur d'autofluorescence corrigée vis-à-vis de différents facteurs de réflexion de tissu cutané sous UV, que peuvent présenter différents sujets respectifs, soit réduite au minimum ou tout au moins diminuée.

2. Procédé selon la revendication 1, dans lequel la première longueur d'onde ou la première plage de longueurs d'onde, se situe dans une plage comprise entre 360 nm et 365 nm, et dans lequel la seconde longueur d'onde ou la seconde plage de longueurs d'onde se situe dans une plage comprise entre 390 nm et 395 nm.

3. Procédé selon la revendication 1, dans lequel la première longueur d'onde ou la première plage de longueurs d'onde, se situe dans une plage comprise entre 620 nm et 650 nm, et dans lequel la seconde longueur d'onde ou la seconde plage de longueurs d'onde se situe dans une plage supérieure à 675 nm.

4. Procédé selon la revendication 1, dans lequel ladite première longueur d'onde ou ladite première plage de longueurs d'onde, se situe dans ladite plage comprise entre 450 nm et 525 nm, dans lequel ladite seconde longueur d'onde ou ladite seconde plage de longueurs d'onde se situe dans une plage supérieure à 620 nm ou à 625 nm.

5. Procédé selon la revendication 1, dans lequel ladite première longueur d'onde ou ladite première plage de longueurs d'onde se situe dans ladite plage comprise entre 450 nm et 525 nm, et dans lequel ladite seconde longueur d'onde ou ladite seconde plage de longueurs d'onde se situe dans ladite plage de longueurs d'onde supérieure à 620 nm, ou selon la revendication 3 ou la revendication 4, dans lequel la seconde longueur d'onde ou la seconde plage de longueurs d'onde est inférieure à 900 nm.

6. Procédé selon l'une quelconque des revendications précédentes, dans lequel la correction se rapporte à un rapport entre le facteur de réflexion à la première longueur d'onde ou dans la première plage de longueurs d'onde, et à la seconde longueur d'onde ou dans la seconde plage de longueurs d'onde.

7. Procédé selon l'une quelconque des revendications précédentes, dans lequel la relation entre le premier facteur de réflexion mesuré à la première longueur d'onde ou dans la première plage de longueurs d'onde, et le second facteur de réflexion mesuré à la seconde longueur d'onde ou dans la seconde plage de longueurs d'onde, est obtenue en déterminant une pente d'une courbe à partir d'un logarithme du premier facteur de réflexion mesuré à la première longueur d'onde ou dans la première plage de longueurs d'onde sur un logarithme du second facteur de réflexion mesuré à la seconde longueur d'onde ou dans la seconde plage de longueurs d'onde.

8. Procédé selon la revendication 7, dans lequel la courbe, dont on détermine la pente, représente, sur une échelle logarithmique, un spectre de réflexion obtenu en provenance du sujet à des longueurs d'onde supérieures à 620 nm.

9. Procédé selon la revendication 1, dans lequel la valeur d'autofluorescence corrigée est obtenue grâce à la formule :

$$AF_{corr} = AF_m + \alpha_1\ MI_1 + \alpha_2\ RedLnSlope + \alpha_3\ Age$$

dans laquelle :

- $AF_{corr}$ représente la valeur d'autofluorescence corrigée ;
- $AF_m$ représente la valeur d'autofluorescence mesurée, à savoir non corrigée ;
- $MI_1$ représente le rapport des valeurs des facteurs de réflexion contre ledit tissu cutané à deux longueurs d'onde différentes qui se situent dans une plage comprise entre 300 nm et 420 nm dudit rayonnement d'excitation, ou une pente dans une échelle logarithmique d'un spectre de réflexion dans ladite plage comprise entre 300 nm et 420 nm ;
- RedLnSlope représente la pente dans une échelle logarithmique d'un spectre de réflexion à des longueurs d'onde supérieures à 620 nm, ou un rapport entre les valeurs des facteurs de réflexion à des longueurs d'onde supérieures à 620 nm ;

- Age représente l'âge du sujet en question ; et

- $\alpha_1$, $\alpha_2$ et $\alpha_3$ sont des coefficients déterminés par exemple par une analyse de régression sur un ensemble de données de sujets.

10. Procédé selon l'une quelconque des revendications précédentes, dans lequel la valeur d'autofluorescence corrigée est obtenue en corrigeant l'autofluorescence mesurée selon un écart de facteur de réflexion sur une plage de longueurs d'onde UV (comprise entre 300 nm et 420 nm) par rapport à une ligne droite.

11. Procédé selon l'une quelconque des revendications précédentes, dans lequel une estimation de la teneur en produits terminaux de glycation avancée (AGE) accumulés dans ledit tissu (peau) du sujet en question, est obtenue sur la base de la valeur d'autofluorescence corrigée.

12. Procédé selon la revendication 11, dans lequel une estimation de la teneur en produits terminaux de glycation avancée (AGE), ou du risque pour la santé du sujet, est obtenue sur la base d'une combinaison de la valeur d'autofluorescence mesurée et de la valeur d'autofluorescence corrigée.

13. Appareil destiné à déterminer la valeur d'autofluorescence d'un tissu cutané d'un sujet, comprenant :

une unité d'acquisition qui comprend des moyens de rayonnement, destinés à irradier *in vivo* et de manière non effractive un tissu cutané intact, derrière une fenêtre d'irradiation particulière avec un rayonnement électromagnétique d'excitation qui présente une longueur d'onde ou une plage de longueurs d'onde à l'intérieur de la plage de longueurs d'onde d'excitation comprise entre 300 nm et 420 nm ;

des moyens de détection destinés à mesurer le rayonnement fluorescent électromagnétique en provenance dudit tissu cutané dans une plage de longueurs comprise entre 420 nm et 600 nm ; et

des moyens de génération destinés à générer une valeur d'autofluorescence mesurée dudit tissu selon ladite quantité mesurée de rayonnement fluorescent en provenance dudit tissu ;

lesdits moyens de détection de rayonnement et de génération étant agencés en outre de façon à obtenir un premier facteur de réflexion mesuré à une première longueur d'onde ou dans une première plage de longueurs d'onde, et un second facteur de réflexion mesuré à une seconde longueur d'onde ou dans une seconde plage de longueurs d'onde, différente de la ladite première longueur d'onde ou de ladite première plage de longueurs d'onde ;

dans lequel lesdites premières et secondes longueurs d'onde ou plages de longueurs d'onde, se situent toutes deux à l'intérieur de la plage de longueurs d'onde d'excitation avec lesquelles le tissu cutané est irradié pour être excité, ou se situent toutes deux à l'intérieur de la plage de longueurs d'onde dans laquelle le rayonnement fluorescent émis est mesuré, ou se situent toutes deux dans une plage de longueurs d'onde supérieure à 620 nm, ou dans lequel ladite première longueur d'onde ou ladite première plage de longueurs d'onde se situe dans une plage de longueurs d'onde comprise entre 450 nm et 525 nm, et ladite seconde longueur d'onde ou ladite seconde plage de longueurs d'onde se situe dans une plage de longueurs d'onde supérieure à 620 nm ; et

lesdits moyens de génération étant agencés de façon à corriger l'autofluorescence mesurée selon une relation entre lesdits premier et second facteurs de réflexion mesurés de telle façon que la dépendance de la valeur d'autofluorescence corrigée vis-à-vis de différents facteurs de réflexion de tissu cutané sous UV, que peuvent présenter différents sujets respectifs, soit réduite au minimum ou tout au moins diminuée.

14. Appareil selon la revendication 13, dans lequel les moyens de rayonnement sont agencés de façon à émettre de manière sélective un rayonnement à deux longueurs d'onde au moins mutuellement distinctes, ou dans des plages de longueurs d'onde, dans une plage de longueurs d'onde supérieure à 420 nm ; et dans lequel les moyens de détection sont agencés de façon à détecter de manière sélective une réflexion à deux longueurs d'onde au moins mutuellement distinctes, ou dans des plages de longueurs d'onde, dans une plage de longueurs d'onde supérieure à 420 nm.

15. Appareil selon la revendication 13 ou la revendication 14, agencé de façon à empêcher l'activation des moyens de rayonnement destinés à émettre le rayonnement d'excitation électromagnétique, à moins qu'aucun rayonnement ne soit détecté par les moyens de détection.

16. Appareil selon la revendication 15, dans lequel les moyens de rayonnement sont adaptés en outre de façon à irradier le tissu cutané derrière la fenêtre d'irradiation avec un rayonnement électromagnétique qui se situe au moins dans une plage de longueurs d'onde supérieure à 420 nm ; et

dans lequel ledit appareil est agencé de façon à empêcher l'activation des moyens de rayonnement destinés à émettre le rayonnement d'excitation électromagnétique, à moins qu'un facteur de réflexion qui satisfait à une référence caractéristique d'une peau à irradier, ne soit détecté par les moyens de détection lorsque la peau est irradiée par les moyens de rayonnement avec ledit rayonnement électromagnétique dans au moins la plage de longueurs d'onde supérieure à 420 nm.

Fig. 1

Fig. 2

Fig. 3

Fig. 4

Fig. 5

Fig. 6

**Fig. 7**

**Fig. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0122869 A

- WO 2005045393 A

**Non-patent literature cited in the description**

- **M. KOETSIER et al.** Skin color independent assessment of aging using skin autofluorescence. Optical Society of America, Inc, 2010
- Ethnic variation in melanin content and composition in photoexposed and photoprotected human skin. **S. ALALUF ; D. ATKINS ; K. BARRETT ; M. BLOUNT ; N. CARTER ; A. HEATH.** Pigment cell research. International Pigment Cell Society, 2002, vol. 15, 112-8
- **R.R. ANDERSON ; J.A. PARRISH.** The optics of human skin. *J Invest Dermatol,* 1981, vol. 77, 13-19
- **L. BACHMANN ; D. ZEZELL ; A. RIBEIRO ; L. GOMES ; A. ITO.** Fluorescence spectroscopy of biological tissues-A review. *Applied Spectroscopy Reviews,* 2006, vol. 41, 575-590
- **G.S. BARSH.** What controls variation in human skin color?. *PLoS Biol,* 2003, vol. 1, E27
- **R. CHEN ; Z. HUANG ; H. LUI ; I. HAMZAVI ; D.I. MCLEAN ; S. XIE ; H. ZENG.** Monte Carlo simulation of cutaneous reflectance and fluorescence measurements--the effect of melanin contents and localization. *J Photochem Photobiol B,* 2007, vol. 86, 219-226
- **J.M. COREMANS ; C. INCE ; H.A. BRUINING ; G.J. PUPPELS.** Semi-)quantitative analysis of reduced nicotinamide adenine dinucleotide fluorescence images of blood-perfused rat heart. *Biophys J,* 1997, vol. 72, 1849-1860
- **J.B. DAWSON ; D.J. BARKER ; D.J. ELLIS ; E. GRASSAM ; J.A. COTTERILL ; G.W. FISHER ; J.W. FEATHER.** A theoretical and experimental study of light absorption and scattering by in vivo skin. *Phys Med Biol,* 1980, vol. 25, 695-709
- **N.C. DEN HOLLANDER ; D.J. MULDER ; R. GRAAFF ; S.R. THORPE ; J.W. BAYNES ; G.P. SMIT ; A.J. SMIT.** Advanced glycation end products and the absence of premature atherosclerosis in glycogen storage disease Ia. *J Inherit Metab Dis,* 2007, vol. 30, 916-923

- **J.W. FEATHER ; M. HAJIZADEH-SAFFAR ; G. LESLIE ; J.B. DAWSON.** A portable scanning reflectance spectrophotometer using visible wavelengths for the rapid measurement of skin pigments. *Phys Med Biol,* 1989, vol. 34, 807-820
- **K.M. KATIKA ; L. PILON.** Steady-state directional diffuse reflectance and fluorescence of human skin. *Appl Opt,* 2006, vol. 45, 4174-4183
- **M. KOETSIER ; H.L. LUTGERS ; A.J. SMIT ; T.P. LINKS ; R.D. VRIES ; R.O. GANS ; G. RAKHORST ; R. GRAAFF.** Skin autofluorescence for the risk assessment of chronic complications in diabetes: a broad excitation range is sufficient. *Opt Express,* 2009, vol. 17, 509-519
- **M. KOETSIER ; H.L. LUTGERS ; C. DE JONGE ; T.P. LINKS ; A.J. SMIT ; R. GRAAFF.** Reference values of skin autofluorescence. *Diabetes Technol Ther,* 2010, vol. 12, 399-403
- **N. KOLLIAS ; A. BAQER.** Spectroscopic characteristics of human melanin in vivo. *J Invest Dermatol,* 1985, vol. 85, 38-42
- **N. KOLLIAS ; A.H. BAQER.** Absorption mechanisms of human melanin in the visible, 400-720 nm. *J Invest Dermatol,* 1987, vol. 89, 384-388
- **N. KOLLIAS ; G. ZONIOS ; G.N. STAMATAS.** Fluorescence spectroscopy of skin. *Vib Spectrosc,* 2002, vol. 28, 17-23
- **H.L. LUTGERS ; E.G. GERRITS ; R. GRAAFF ; T.P. LINKS ; W.J. SLUITER ; R.O. GANS ; H.J. BILO ; A.J. SMIT.** Skin autofluorescence provides additional information to the UK Prospective Diabetes Study (UKPDS) risk score for the estimation of cardiovascular prognosis in type 2 diabetes mellitus. *Diabetologia,* 2009, vol. 52, 789-797
- **C. MAGNAIN ; M. ELIAS ; J. FRIGERIO.** Skin color modeling using the radiative transfer equation solved by the auxiliary function method. *Journal of the Optical Society of America. A, Optics, image science, and vision,* 2007, vol. 24, 2196-205

- **T. MATSUMOTO ; T. TSURUMOTO ; H. BABA ; M. OSAKI ; H. ENOMOTO ; A. YONEKURA ; H. SHINDO ; T. MIYATA.** Measurement of advanced glycation endproducts in skin of patients with rheumatoid arthritis, osteoarthritis, and dialysis-related spondyloarthropathy using non-invasive methods. *Rheumatol Int,* 2007, vol. 28, 157-160
- **R. MEERWALDT ; R. GRAAFF ; P.H. OOMEN ; T.P. LINKS ; J.J. JAGER ; N.L. ALDERSON ; S.R. THORPE ; J.W. BAYNES ; R.O. GANS ; A.J. SMIT.** Simple non-invasive assessment of advanced glycation endproduct accumulation. *Diabetologia,* 2004, vol. 47, 1324-1330
- **R. MEERWALDT ; J.W. HARTOG ; R. GRAAFF ; R.J. HUISMAN ; T.P. LINKS ; N.C. DEN HOLLANDER ; S.R. THORPE ; J.W. BAYNES ; G. NAVIS ; R.O. GANS.** Skin autofluorescence, a measure of cumulative metabolic stress and advanced glycation end products, predicts mortality in hemodialysis patients. *J Am Soc Nephrol,* 2005, vol. 16, 3687-3693
- **M. MONAMI ; C. LAMANNA ; F. GORI ; F. BARTALUCCI ; N. MARCHIONNI ; E. MANNUCCI.** Skin autofluorescence in type 2 diabetes: beyond blood glucose. *Diabetes Res Clin Pract,* 2008, vol. 79, 56-60
- **D.J. MULDER ; P.L. VAN HAELST ; S. GROSS ; K. DE LEEUW ; J. BIJZET ; R. GRAAFF ; R.O. GANS ; F. ZIJLSTRA ; A.J. SMIT.** Skin autofluorescence is elevated in patients with stable coronary artery disease and is associated with serum levels of neopterin and the soluble receptor for advanced glycation end products. *Atherosclerosis,* 2008, vol. 197, 217-223
- **D.J. MULDER ; T.V. WATER ; H.L. LUTGERS ; R. GRAAFF ; R.O. GANS ; F. ZIJLSTRA ; A.J. SMIT.** Skin autofluorescence, a novel marker for glycemic and oxidative stress-derived advanced glycation endproducts: an overview of current clinical studies, evidence, and limitations. *Diabetes Technol Ther,* 2006, vol. 8, 523-535
- **R. NA ; I.M. STENDER ; M. HENRIKSEN ; H.C. WULF.** Autofluorescence of human skin is age-related after correction for skin pigmentation and redness. *J Invest Dermatol,* 2001, vol. 116, 536-540
- **K.P. NIELSEN ; L. ZHAO ; G.A. RYZHIKOV ; M.S. BIRYULINA ; E.R. SOMMERSTEN ; J.J. STAMNES ; K. STAMNES ; J. MOAN.** Retrieval of the physiological state of human skin from UV-Vis reflectance spectra - a feasibility study. *J Photochem Photobiol B,* 2008, vol. 93, 23-31
- **I. NISHIDATE ; Y. AIZU ; H. MISHINA.** Estimation of melanin and hemoglobin in skin tissue using multiple regression analysis aided by Monte Carlo simulation. *Journal of biomedical optics,* 2004, vol. 9, 700-10
- **J. SANDBY-MØLLER ; T. POULSEN ; H.C. WULF.** Influence of epidermal thickness, pigmentation and redness on skin autofluorescence. *Photochem Photobiol,* 2003, vol. 77, 616-620
- Reflectance and fluorescence spectroscopy of human skin in vivo. **Y.P. SINICHKIN ; N. KOLLIAS ; G.I. ZONIOS ; S.R. UTZ ; V.V. TUCHIN.** Handbook of optical biomedical diagnostics. SPIE Press, 2002, 725-785
- **G.N. STAMATAS ; R.B. ESTANISLAO ; M. SUERO ; Z.S. RIVERA ; J. LI ; A. KHAIAT ; N. KOLLIAS.** Facial skin fluorescence as a marker of the skin's response to chronic environmental insults and its dependence on age. *Br J Dermatol,* 2006, vol. 154, 125-132
- **H. UENO ; H. KOYAMA ; S. TANAKA ; S. FUKUMOTO ; K. SHINOHARA ; T. SHOJI ; M. EMOTO ; H. TAHARA ; R. KAKIYA ; T. TABATA.** Skin autofluorescence, a marker for advanced glycation end product accumulation, is associated with arterial stiffness in patients with end-stage renal disease. *Metabolism,* 2008, vol. 57, 1452-1457
- **W.G. ZIJLSTRA ; A. BUURSMA ; O.W. VAN ASSENDELFT.** Visible and near infrared absorption spectra of human and animal haemoglobin. *Zeist, The Netherlands: VSP BV,* 2000
- **G. ZONIOS ; A. DIMOU.** Modeling diffuse reflectance from semi-infinite turbid media: application to the study of skin optical properties. *Opt Express,* 2006, vol. 14, 8661-8674
- **G. ZONIOS ; A. DIMOU ; I. BASSUKAS ; D. GALARIS ; A. TSOLAKIDIS ; E. KAXIRAS.** Melanin absorption spectroscopy: new method for noninvasive skin investigation and melanoma detection. *J Biomed Opt,* 2008, vol. 13, 14017
- **G. ZONIOS ; A. DIMOU.** Melanin optical properties provide evidence for chemical and structural disorder in vivo. *Opt Express,* 2008, vol. 16, 8263-8268